# EUROPEAN PATENT APPLICATION

(11) **EP 1 101 462 A1**
(43) Date of publication of application: **23.05.2001**
(21) Application number: 00125055.4
(22) Date of filing: 17.11.2000
(51) Int. Cl.: A61F 5/441

(54) **Ostomy pouches**

(30) Priority: 17.11.1999 GB 9927135
(71) Applicant: Wood, Michael, Stockport, Cheshire SK7 1PB (GB)
(72) Inventor: Wood, Michael, Stockport, Cheshire SK7 1PB (GB)
(74) Representative: Ajello, Michael John

(57) **Abstract**

An ostomy pouch used by colostomy and ileostomy patients to receive bodily waste from a stoma the pouch comprises a bag (10), an inlet aperture (12) surrounded by an adhesive attachment collar (11), and a valve assembly (17) for venting gas from the bag. The valve means comprises an outer resilient frusto-conical diaphragm (18) integrally formed with a resilient inner cylindrical core (19) having a slot (20) cut in its wall. By squeezing the outer diaphragm (18) the inner core (19) is distorted to cause the slot (20) to open and release gas from within the bag, through a filter (15) to atmosphere. The valve assembly (17) enables the bag to be vented at will and thus provides user control to prevent ballooning and pancaking of the bag.

## Description

THIS INVENTION concerns pouches used by colostomy and ileostomy patients, to receive bodily waste from a stoma.

Such devices are usually disposable but, in use, the escape of odorous gases from the stoma into the pouch is unpredictable and cannot normally be controlled by the patient's natural means.

Gas entering the ostomy pouch causes it to inflate. This is known by the term "ballooning". Excessive ballooning can result in the patient's clothing becoming distorted which in turn causes discomfort and personal embarrassment accompanied by a feeling of social isolation and loss of confidence and dignity.

In some ostomy systems currently in use, ballooning is minimised by the inclusion of permanently open exhaust vents in conjunction with filters to eliminate or reduce odour. However, a problem is experienced with this type of device in that the permanently open vent allows the continuous uncontrolled exhausting of gases, which can result in the complete deflation of the pouch and this is known as "pancaking". This can cause the walls of the pouch to stick together and/or the outer wall of the pouch to adhere to the patient's stoma, and impede the passage of waste substances into the pouch.

In some examples, ballooning and pancaking are controlled by the use of vent cover labels which can be removed or remain in place as the patient pleases thus to determine whether to allow or prevent the continuous venting of the pouch. Such labels can be removed temporarily for exhausting and then replaced but for this purpose the patient may need to remove or undo clothing and so this must be carried out in private, and there exists the additional problem that the label once removed may not properly re-seal the exhaust aperture.

Other methods adopted by patients include partial removal of the pouch which may then not be properly resealed with the body, thus carrying the risk of leakage.

Some patients will pierce an inflated pouch with a pin or other sharp object to release the pressure and then reseal it with adhesive tape. These method are inconvenient, uncontrolled and often result in excessive stress for the patient.

Therefore, the patient is often faced with having to tolerate either ballooning or pancaking because of the absence of variable and selective control.

Patent specification CA 2145586 describes a mechanical venting assembly which may be attached to an ostomy pouch and can be operated by the patient, but this involves engineered parts which are both cumbersome and expensive to produce.

It is an object of the present invention to provide an ostomy pouch with a self-contained venting assembly which is manufactured with the pouch, is compact and easy to use and occupies minimal space thus to be more or less invisible within the patient's clothing.

The venting facility is normally closed providing a fluid-tight seal so that unwanted and indiscriminate escape of gases is prevented thus eliminating personal embarrassment but providing for the patient a positive variable control over the release of gas when and where required thus to prevent ballooning and pancaking of the ostomy pouch.

According to the present invention, there is provided an ostomy pouch comprising a bag, an opening in the wall of the bag with a sealing collar for attachment over a stoma and valve means for venting gas from the bag; characterised in that the valve means includes a resilient member having a normally-closed gas escape aperture, and means to permit temporary distortion of the resilient member selectively to open the gas escape aperture; and in that resilient recovery of the resilient member re-closes the gas escape aperture.

The valve means may include a resilient outer diaphragm integrally formed with an inner cylindrical core defining a normally closed gas escape slot therein such that inwards distortion of the outer diaphragm causes local distortion of the inner cylindrical core sufficient to cause the slot to open and permit the passage of gas therethrough, the inner core communicating with atmosphere and, when the slot is open, with the interior of the bag.

An odour absorbing filter may be positioned between the interior of the bag and the valve means such that all gas exhausted from the bag must pass through said filter.

The valve means is preferably out of direct alignment with said opening and sealing collar.

The outer diaphragm may be of frusto-conical shape and of a plastics material and thermally welded to the outer wall of the bag around an outlet aperture therein.

The inner core and outer diaphragm may be integrally moulded from a resilient plastics material.

An embodiment of the invention will now be described, by way of example, with reference to the accompanying drawings, in which:-
Fig. 1 is a cross-sectional view of an ostomy pouch having gas venting means in accordance with the invention;
and Fig. 2 is an enlarged cross-sectional view of a valve means being part of the gas venting means.

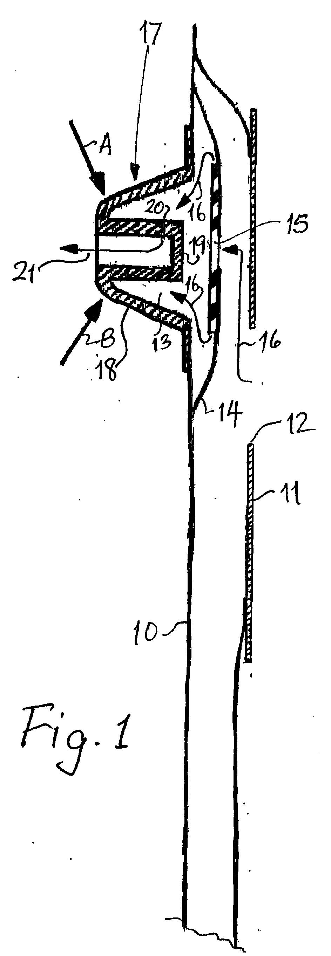

Referring now to Fig. 1, there is shown an ostomy bag 10 having an attachment collar 11 with an adhesive outer surface for attachment to a patient's body around a stoma. The collar 11 has an inlet aperture 12 for the passage into the bag 10 of bodily waste.

As with most ostomy pouches the entire device is disposable after a period of use.

In accordance with the invention, the bag 10 contains an outlet aperture 13 covered internally by an inner skin 14 containing an odour filter 15 such that all gases passing to the outlet aperture 13 do so through the filter 15 as indicated by arrows 16.

Welded or bonded to the outer wall of the bag 10 around the aperture 13 is a valve assembly generally indicated at 17. This consists of an outer frusto-conical diaphragm 18 of a resilient plastics material, formed integrally with an inner cylindrical core 19 having, in one region of the cylindrical wall thereof a cut or slot 20 which is normally closed to prevent the passage of gas. The interior of the core 19 communicates with atmosphere for the escape of gas as illustrated by the arrow 21.

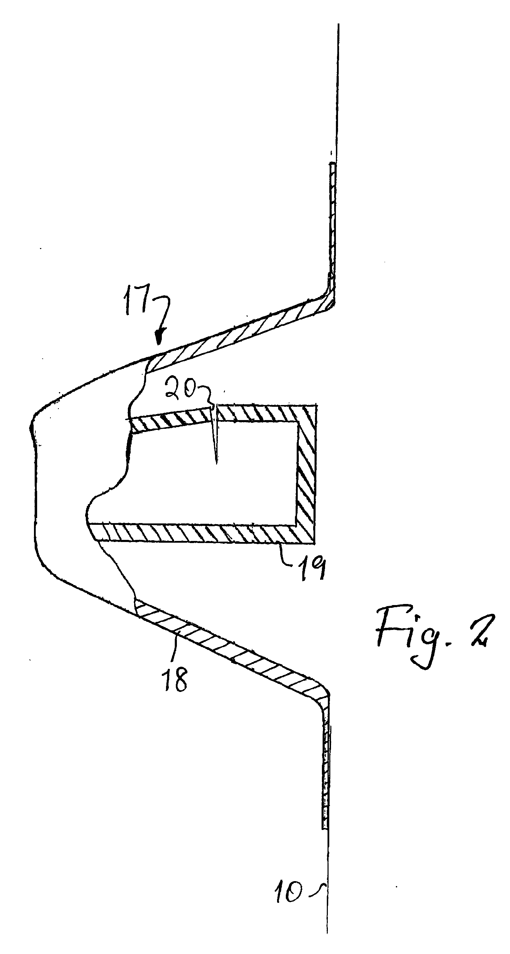

The slot 20 is normally closed, but the resilient nature of the valve assembly is such that by squeezing the outer diaphragm 18 at positions A and B, and this is illustrated clearly in Fig. 2, the outer end of the core 19 may become distorted such as to cause the slot 20 to open whereby gases which have passed outwardly through the filter 15 may escape to atmosphere.

Once the diaphragm 18 is released the natural resilience and elasticity of the material cause it and the core 19 to reform or recover whereby the slot 20 closes and prevents the further passage of gas either into or out of the bag 10. Any increase of pressure within the bag maintains the slot 20 in a closed and sealed condition.

Thus, a patient wearing an ostomy pouch of this kind may, when and where appropriate, allow the venting of gases from the pouch thus to prevent ballooning. However, since the venting aperture is normally closed and thus continuous uncontrolled venting is prevented, the pouch may remain lightly inflated by the contained gas to prevent pancaking.

The patient may vent the bag and of course will usually do so where such action does not cause embarrassment, and thus the patient has complete control over the presence of gas within the pouch and retains complete confidence and dignity in the use of an ostomy pouch. The venting system is easy to control by simple fingertip operation and is of uncomplicated and relatively inexpensive construction. It is readily compatible with current manufacturing methods and materials, and is disposable along with the pouch and thus requires no cleaning.

## Claims

1. An ostomy pouch comprising a bag, an opening in the wall of the bag with a sealing collar for attachment over a stoma and valve means for venting gas from the bag; characterised in that the valve means includes a resilient member having a normally-closed gas escape aperture, and means to permit temporary distortion of the resilient member selectively to open the gas escape aperture; and in that resilient recovery of the resilient member re-closes the gas escape aperture.

2. An ostomy pouch according to Claim 1, wherein the resilient member includes a resilient outer part integrally formed with a resilient inner part defining a normally closed gas escape slot therein such that inwards distortion of the resilient outer part causes local distortion of the resilient inner part sufficient to cause the slot to open and permit the passage of gas therethrough, the resilient inner part communicating with atmosphere and, when the slot is open, with the interior of the bag.

3. An ostomy pouch according to any preceding claim, including an odour absorbing filter positioned between the interior of the bag and the valve means such that all gas exhausted from the bag must pass through said filter.

4. An ostomy pouch according to any preceding claim, wherein the valve means is out of direct alignment with said opening and sealing collar.

5. An ostomy pouch according to Claim 2, wherein the resilient outer part is of frusto-conical shape and of a plastics material and thermally welded to the outer wall of the bag around an outlet aperture therein.

6. An ostomy pouch according to Claim 2, wherein the resilient inner part is a cylindrical core which, with the resilient outer part, is integrally moulded from a resilient plastics material.

7. An ostomy pouch according to Claim 4, wherein the gas escape slot is formed as a cut in the cylindrical wall of the cylindrical core.

8. An ostomy pouch according to any preceding claim, wherein the normally closed gas escape aperture is so- formed and located that any increase of pressure within the bag maintains the aperture in a closed and sealed condition.
